Europäisches Patentamt

European Patent Office

Office européen des brevets

(19)

(11) **EP 0 898 470 B1**

(12) **EUROPÄISCHE PATENTSCHRIFT**

(45) Veröffentlichungstag und Bekanntmachung des Hinweises auf die Patenterteilung:
**03.01.2001 Patentblatt 2001/01**

(21) Anmeldenummer: **97923740.1**

(22) Anmeldetag: **21.04.1997**

(51) Int Cl.⁷: **A61F 2/34**

(86) Internationale Anmeldenummer:
**PCT/DE97/00793**

(87) Internationale Veröffentlichungsnummer:
**WO 97/39702 (30.10.1997 Gazette 1997/46)**

(54) **HÜFTGELENKPFANNE MIT SPEZIALGEWINDE**

HIP JOINT GLENOID CAVITY WITH A SPECIAL THREAD

CAVITE GLENOIDE DE L'ARTICULATION DE LA HANCHE AVEC UN FILET SPECIAL

(84) Benannte Vertragsstaaten:
**AT BE CH DE ES FR GB IT LI NL SE**

(30) Priorität: **22.04.1996 DE 19615876**

(43) Veröffentlichungstag der Anmeldung:
**03.03.1999 Patentblatt 1999/09**

(73) Patentinhaber: **Hörmansdörfer, Gerd**
**D-31303 Burgdorf (DE)**

(72) Erfinder: **Hörmansdörfer, Gerd**
**D-31303 Burgdorf (DE)**

(56) Entgegenhaltungen:
EP-A- 0 237 751          EP-A- 0 358 345
EP-A- 0 586 335          EP-A- 0 667 134
WO-A-85/00284           WO-A-95/15132
WO-A-95/18586           DE-A- 3 840 468
DE-A- 4 408 527          DE-C- 3 535 959
DE-C- 3 701 381          DE-U- 9 408 200

## Beschreibung

Die Erfindung betrifft eine Hüftgelenkpfanne mit einem Spezialgewinde, wie in Anspruch 1 beschrieben, welche zur Verwendung als Teil des künstlichen Gelenkersatzes beim Menschen vorgesehen ist.

Derartige Hüftgelenkpfannen mit Gewinde gehören zu der Gruppe der sogenannten Schraubpfannen, da sie in das knöcherne Lager entweder nach einem Vorschnitt mittels Werkzeugs oder selbstschneidend einschraubbar sind. In dieser Gruppe sind hinsichtlich der Mantelform des Schalenkörpers z.B. hypo-, hemi-, hyper- oder parasphärische, konische, konischsphärische, parabolische, toroidische, ellipsoidische und ähnliche Geometrien bekannt.

Hinsichtlich der Gestaltung des auf dem Schalenkörper liegenden Gewindes besteht eine gewisse Bandbreite, z.B. in Bezug auf die Gestaltung des Gewindeprofils aus Gewindezahn und -rille sowie der Steigung und der überdeckten Erstreckung. Es hat sich inzwischen die Erkenntnis durchgesetzt, daß eine Aufgliederung des Gewindeprofils in schmale Gewindezähne und breite Gewinderillen ähnlich wie bei einem Holzschraubengewinde vorteilhaft ist, weil es das Festigkeitsverhältnis zwischen dem Werkstoff Metall und dem Knochenmaterial berücksichtigt und wegen des günstigeren Breiten/Höhenverhältnisses der zwischen den Gewindezähnen verbleibenden Knochenrippe eine Erhaltung der Knochensubstanz unterstützt.

In Fachkreisen wird noch die Frage diskutiert, ob bezüglich der Form des Gewindezahns ein sogenanntes Flachgewinde mit schmalem rechteckigen Querschnitt oder ein schlanker dreieckiger bzw. trapezförmiger Gewindezahn die bessere Wahl ist. Die entsprechende Bewertung muß einerseits den Kraftbedarf während des Einschneidvorgangs und andererseits die erzielbare Kurz- und Langzeitfixation berücksichtigen. Für beide Gewindearten gilt, daß zur Erzielung eines nur geringe Kräfte erfordernden Einschneidvorgangs im Gewindezug der Schraubpfanne Schneidzähne mit einem sogenannten positiven Spanwinkel vorhanden sein müssen. Je nach Anzahl der Spannuten und der damit gebildeten Schneidzähne und der Zahngeometrie kann es erforderlich sein, einen hinter dem jeweiligen Schneidzahn liegenden Freiwinkel zu realisieren, um einen zu starken Klemmeffekt während des Einschneidvorgangs zu vermeiden. Beim Flachgewinde ist es üblich, zu diesem Zweck die umlaufende Kontur im Zahnkopfbereich zu hinterschneiden.

Die technischen Anstrengungen der letzten Jahre haben vor allem darauf abgezielt, die vorzeitige Auslockerung solcher Hüftgelenkpfannen zu reduzieren. Dem verständlichen Ziel einer beschwerdefreien Benutzungsdauer bis zur Lebenserwartung des Patienten ist man schrittweise näher gekommen, indem das Gewindeprofil optimiert, der Gewindegrund besser der Kontur des Schalenmantels angepaßt, und die Oberfläche rauh strukturiert wurde. Trotzdem besteht weiterhin die Aufgabe, durch entsprechende Feinarbeit derartige Implantate weiter zu verbessern, um das angestrebte Ziel zu erreichen. Dabei wird neben einer noch gleichmäßigeren Kraftübertragung vom Implantat auf das Acetabulum insbesondere eine deutliche Reduzierung der Einschraubkräfte beim Implantieren und eine sehr gute Fühlbarkeit derjenigen Position gefordert, in welcher der Schalenmantel der Schraubpfanne im Knochengrund zur Auflage kommt. Damit wären die Gefahren des ungenügend tiefen Einsetzens bzw. des Überdrehens des Implantats besser beherrschbar.

Die beschriebene Aufgabe wird nach der Erfindung durch eine Schraubpfanne mit den Merkmalen gemäß dem Anspruch 1 gelöst. Dabei wird die in axialer Richtung gemessene Steigung des auf dem Schalenmantel abgewickelten Gewindes in Abhängigkeit von der Krümmung der Axialkontur des Schalenmantels, des Zahnprofils und seines Kippwinkels, dem Drallwinkel der Spannut und der Rauhigkeit der Oberfläche aufgrund der sich dadurch beim Implantieren oder Vorschneiden ergebenden Schnittkraftverteilung abweichend vom üblichen konstanten Verlauf variiert, um eine Minimierung des Einschraubkraftbedarfs zu bewirken. Mittels einer entsprechenden Steigungsvariation wird der Kraftaufwand für das Einschrauben der Hüftgelenkpfanne merklich reduziert und dabei die Gefahr des vorzeitigen Verklemmens bzw. der Beckensprengung beseitigt. Dabei wird der Aufsetzpunkt des Schalenmantels der Schraubpfanne auf die knöcherne Lagerfläche durch einen starken Drehmomentsprung beim Einschrauben sehr gut fühlbar, wodurch das ständige Abnehmen des Aufsatzschlüssels und die optische bzw. mechanische Kontrolle praktisch entfallen. Auch bei einer in sehr seltenen Fällen möglicherweise einmal auftretenden Pfannenlockerung ist nach dem Herausschrauben des Implantats lediglich mit einer relativ geringen Zerfurchung des beteiligten Knochensaums zu rechnen, sodaß das knöcherne Lager mittels einer sehr knochensparenden Ausschälung für die neuerliche Implantation vorbereitet werden kann.

Gleichzeitig wird mit der Erfindung vorgeschlagen, das in den obigen Komplex einbezogene Gewindeprofil unter Berücksichtigung der später unter bestimmungsgemäßer Belastung auftretenden Kraftübertragung vom Implantat auf das knöcherne Lager in Richtung zur Pfannenkappe bzw. zum Pfannenpol hin, vorzugsweise weiter als bisher üblich zu kippen. Durch diese Gewindegestaltung werden die Kraftübertragungsverhältnisse zwischen dem erfindungsgemäßen Implantat und dem knöchernen Lager verbessert und sowohl die Kippstabilität als auch die Fixation erhöht, indem in den Gewinderillen einseitig auftretende Lastspitzen reduziert, bzw. die Summe aus statischer und dynamischer Last gleichmäßiger und auf eine größere Fläche verteilt wird.

Spezielle Ausgestaltungen und Weiterbildungen der Erfindung, sowie die Vorgehensweisen zur praktischen konstruktiven Gestaltung des Gewindes und der Steigungsvariation sind den Unteransprüchen zu entnehmen.

Der Erfindung liegt die Erkenntnis zugrunde, daß an den drei freien Flächen des Gewindezahnes einer Schraubpfanne, namentlich an der äquator-, kopf- und polseitigen Fläche (die Lagebezeichnung "Äquator" wird im folgenden der Einfachheit halber für alle Schalengeometrien benutzt, obwohl sie im strengen Sinne nur für die hemisphärische Schalenform gilt), sowohl während des eigentlichen Einschraubvorgangs beim Implantieren als auch bei der bestimmungsgemäßen Benutzung ungleiche Kräfte auftreten. Mit der Erfindung wird vorgeschlagen, die Gestalt bzw. die Anordnung des Gewindezuges auf der Schraubpfanne unter Berücksichtigung dieser Kräfte auszuführen und zu diesem Zwecke insbesonders eine mehr oder weniger fließend sich ändernde Gewindesteigung heranzuziehen.

Ein sehr einfacher Schritt zur Anwendung der Erfindung besteht darin, herkömmliche Schraubpfannen mit einem ansonsten mit konstanter Steigung geschnittenen Gewinde lediglich im polseitigen Gewindeanstiegsbereich zwischen der Zahnhöhe Null und der vollen Zahnhöhe mit einer anderen Steigung der Zahnflanken herzustellen, um die auf das auftauchende Zahnprofil wirkenden Kräfte gezielt zu verteilen. Dieser Gewindeanfang stellt bei konventionell hergestellten Schraubpfannen auf der polseitigen Gewindezahnflanke das Relikt eines ringförmigen Ausschnitts einer Plan- oder Kegelfläche dar, welche aus der drehtechnischen Vorbearbeitung resultiert. Da diese Teilfläche relativ schräg zur Einschraubrichtung angeordnet ist, tritt dort ein höherer Einschraubwiderstand auf. Durch ein angepaßtes polwärts gerichtetes Verschwenken dieses Gewindeabschnitts lassen sich die Einschraubkräfte gleichmäßiger verteilen und so vermindern. Für die z.B. drehtechnische Herstellung wird vorgeschlagen, das die polseitige Flanke schneidende Werkzeug im genannten Bereich mit einer gegenüber der sonstigen Gewindesteigung kleineren Steigung, bzw. das die äquatorseitige Flanke schneidende Werkzeug mit einer größeren Steigung zu verfahren.

Eine deutlich anspruchsvollere Umsetzung der Erfindung besteht darin, das Profil des Gewindezahnes aus der üblichen neutralen Lage in Richtung zum Pfannenpol hin zu kippen, bzw. mehr als üblich zu kippen. Bei der überwiegenden Zahl der heute bekannten Schraubpfannen wird auf eine neutrale Zahnstellung zurückgegriffen, das heißt, daß die jeweilige Mittelachse des Gewindezahns in der axialen Schnittebene senkrecht zur Pfannenachse steht. Beim herkömmlichen Flachgewinde verlaufen die beiden Zahnflanken dann entsprechend parallel und beidseitig im gleichen Abstand zu dieser Mittelachse, während bei Schraubpfannen mit Spitzgewinde diese Mittelachse die Winkelhalbierende des von den beiden Zahnflanken eingeschlossenen Winkels darstellt. In der Gruppe der Schraubpfannen mit Spitzgewinde sind auch Modelle mit einem in Richtung zum Äquator hin geneigten Profil bekannt, zum Beispiel das ehemals von der Firma MECRON hergestellte Modell MECRING, oder das Modell ACCUPATH von der Firma HOWMEDICA. Ausgesprochen selten sind Schraubpfannen mit einem in Richtung zum Pfannenpol hin gekippten Zahn, hier sind die Modelle ULTIMA von der Firma JOHNSON & JOHNSON und MÜNCHNER RING der Firma AESCULAP bekannt. Bei der ersteren der beiden genannten Modelle beträgt der Kippwinkel der Mittelachse lediglich 4°, bei der letzteren ist das Zahnprofil so weit gekippt, daß der polseitige Seitenwinkel 0° beträgt. Es sind jedoch keine Schraubpfannen mit im Axialschnitt gekrümmter Kontur des Schalenmantels und geradflankigen Gewindezähnen bekannt, bei denen auch der polseitige Seitenwinkel des Gewindeprofils zum Pfannenpol hin über diesen Wert von 0° hinaus gekippt ist.

In der Deutschen Patentschrift DE 37 01 381 C1 wird eine Schraubpfanne vorgestellt, deren im Querschnitt sichelförmige Gewindezähne zumindest mit ihrem Kopfbereich zum Pfannenpol hin geneigt sind und mit ihren Spitzen entlang eines imaginären einhüllenden Zylinders verlaufen. Bei diesem Konzept muß die gesamte für den Einschraubvorgang erforderliche Zerspanungsarbeit von nur ein bis zwei Schneidkanten am polseitigen Gewindeanfang erbracht werden, was in der Praxis nicht umsetzbar ist. Problematisch ist ferner das Profilwandern beim Einschrauben durch das nicht völlig kongruente Zahnprofil, wodurch zwangsläufig Spiel zwischen dem Gewindezahn und der in den Knochen geschnittenen Gewinderille entstehen muß. Die genannten Mängel mögen wohl auch der Grund dafür sein, daß eine derartige Pfanne als Produkt nicht am Markt erschienen ist.

Eigene Berechnungen des Anmelders zeigen, daß die Beanspruchungen der in die Gewinderillen einer Schraubpfanne eintauchenden Knochenrippen sehr stark von den geometrischen Gegebenheiten abhängig sind. Betrachtet man einmal den Hauptbelastungsbereich ungefähr senkrecht oberhalb der implantierten Schraubpfanne, so zeigt sich, daß zur Erzielung eines kräftemäßigen Minimums bei gleichzeitig homogener Kraftverteilung ein Kippwinkel des Gewindezahns im Bereich von immerhin 45° in Richtung zum Pfannenpol hin erforderlich ist, ein Wert, welcher von keiner der heute auf dem Markt befindlichen Schraubpfannen auch nur annähernd erreicht wird. Bei einem zunehmenden Wegkippen des Gewindezahnes in die neutrale Lage (Mittelachse des Gewindezahns in der Radialebene der Schraubpfanne) zeigen die rechnerischen Ergebnisse des Modells eine überproportional ansteigende Ungleichbelastung des beteiligten Knochenmaterials, wobei auch die Beanspruchungswerte ihrer Höhe nach größer werden.

Auch eine Analyse der bezüglich einer Lastübertragung bestehenden geometrischen Verhältnisse im orthogonal zur Hauptbelastungsrichtung in Richtung zur Körpermitte liegenden Schraubpfannenbereich belegt, daß hier eine Kippung des Zahnprofils in Richtung zum Pfannenpol Vorteile mit sich bringt. Durch diese Maßnahme wird nämlich mit ansteigendem Kippwinkel der Anteil der Scherung an der komplexen Kraftübertragung auf die eintauchende Knochenrippe zurückgedrängt und in zunehmendem Maße durch günstigere Biege- bzw. noch günstigere Druckkräfte ersetzt.

Neben diesen Argumenten, welche aus rein medizintechnischer Sicht für die hiermit vorgeschlagene Kippung des Gewindeprofils sprechen, besteht damit gleichzeitig noch der Vorteil der erleichterten Herstellung, weil sich dadurch im polnahen Zwickel zwischen äquatorseitiger Gewindezahnflanke und Schalenmantel nicht so spitze Winkel ergeben. Deshalb sind für die spanende Fertigung breitere und damit standfestere Wendeschneidplatten verwendbar.

Ein Gewinde der vorgeschlagenen Art kann nicht ohne weiteres mit der für eine Schnittaufteilung erforderlichen Zustellung in herkömmlicher Weise geschnitten werden. Es hat zusätzlich bei der Verwendung für die meisten Schraubpfannengeometrien den schwerwiegenden Nachteil, daß es entweder gar nicht einschraubbar ist oder aber das knochenseitige Zahnbett in Überbreite zerstört, bzw. sehr hohe Einschraubdrehmomente erfordert. Diese nicht hinnehmbaren Mängel sind zwangsläufig mit dem üblichen, in Axialrichtung konstanten Verlauf der Gewindesteigung verbunden. Hier setzt nun die Erfindung an, indem vorgeschlagen wird, die Steigung des Gewindes in einer von der Schnittkraftverteilung bestimmten Gesetzmäßigkeit entlang seiner Erstreckung so zu variieren, daß die oben genannten Nachteile beseitigt und die weiter oben genannten Vorteile nutzbar gemacht werden können.

Die Vorgehensweise zur Festlegung des erfindungsgemäßen Spezialgewindes soll zum besseren Verständnis in einzelnen Schritten erläutert werden. Der erste Schritt besteht darin, in dem halbseitigen Axialschnitt der Schraubpfanne die einzelnen Zähne des Gewindezuges auf dem Schalenmantel so anzuordnen, daß die Projektionszentren der jeweiligen Zahnköpfe auf geraden Äquidistanten, also auf parallel und in gleichem Abstand zueinander verlaufenden geraden Linien zu liegen kommen. Im Falle von echten Spitzgewinden liegt das genannte Projektionszentrum dabei exakt auf der Zahnkopfspitze, während es bei einem schlanken trapezförmigen Zahn auf dem Schnittpunkt der verlängerten Flankenlinien liegt. Bei einem Flachgewinde wird als Projektionszentrum die Mitte der Stirnfläche des Gewindezahnkopfes herangezogen. Die einzelnen Linien der so gebildeten Linienschar sollen im folgenden als Projektionslinien bezeichnet werden. Die zunächst winkelmäßig unkorrigierten Projektionslinien verlaufen dabei als Mittellinien bzw. Winkelhalbierende des jeweiligen Gewindezahns in der Axialschnittebene.

Für die endgültige winkelmäßige Festlegung dieser Projektionslinien wird erfindungsgemäß ein Korrekturwinkel eingeführt, dessen Wert an die angestrebte Schnittkraftverteilung zwischen den beiden Flanken des Gewindezahns während des Einschraubvorgangs anpaßbar ist. Der Korrekturwinkel wird maximal auf den halben zwischen den Zahnflanken eingeschlossenen Winkel begrenzt, sodaß die korrigierte Pojektionslinie mit der Bandbreite ihres positiven oder negativen Korrekturwinkels zwischen der pol- und der äquatorseitigen Zahnflanke um das Projektionszentrum als Drehpunkt schwenkbar ist.

Die oben angesprochenen Schnittkräfte hängen mit dem Einschneidvorgang der Gewindezähne während des Einschraubens der Schraubpfanne zusammen. Sie setzen sich zusammen aus den tatsächlichen Schneidkräften welche von den Schneidkanten der Gewindezähne zu erbringen sind, den Raspelkräften aufgrund einer aufgerauhten Oberfläche, und Quetsch- bzw. Verdrängungskräften durch bestimmte Unzulänglichkeiten der Gewindeanordnung und die Nachgiebigkeit des beteiligten knöchernen Saumes. Es wird nun angestrebt, mittels einer entsprechend angepaßten Variation der Gewindesteigung diese komplexen Schnittkräfte auf ein Minimum zu reduzieren, damit ein zügiges weiches Einschrauben des Implantats auch in härtere Knochenstrukturen erzielbar ist.

Die oben genannten Schneidkanten werden im Gewinde von künstlichen Hüftgelenkpfannen durch Schlitzen der Gewinderippen und Bildung von Spannuten erzeugt. Eine hohe Zahl von Schlitzen und damit Schneidkanten ist gleichbedeutend mit einer geringeren spezifischen Schneidkraftbelastung der einzelnen Schneidkanten. In der Regel ist damit ein generell abgesenkter Schneidkraftbedarf verbunden. Der Schneidkraftbedarf ist ferner noch durch den Spanwinkel, die Schärfe der Schneide, und den sogenannten Freiwinkel beeinflußbar.

Bei den echten Flachgewinden ist die eigentliche Schneidkante am Zahnkopf gebildet. Mittels einer schräg eintauchenden Schlitzung ist hier ein leicht positiver Spanwinkel realisierbar. Gewöhnlich werden solche Flachgewinde im Zahnkopfbereich hinter der Schneidkante hinterfräst, um den angestrebten Freiwinkel zu verwirklichen. Die Gewindeschlitzung ist dabei im allgemeinen relativ neutral, also ohne merklichen Drall. In diesem Fall werden die reinen Schneidkräfte praktisch ausschließlich an der Kopfseite des Gewindezahnes erbracht, während die Flanken bei glatter Oberfläche quasi unbeteiligt sind. An den Flanken können allerdings Raspelkräfte auftreten, wenn die Gewinderippe z.B. mittels einer Sandstrahlung aufgerauht ist. Für diesen Fall wird vorgeschlagen, die üblicherweise parallel laufenden Zahnflanken des Flachgewindes mit einem ganz kleinen konischen, sich zum Zahnkopf verjüngenden Winkel zu versehen, um auch nach dem Einschrauben einen festen Sitz zu garantieren. Auch wenn dann von den Zahnflanken je nach Gestaltung gewisse Raspelkräfte übertragen werden, so sind diese jedoch prinzipiell symmetrisch ausgeglichen. Daraus folgt, daß ein Korrekturwinkel für die weiter oben beschriebene Projektionslinienschar nicht erforderlich ist. Dies gilt im übrigen auch für sogenannte Spitzgewinde und ihre Abwandlungen für den Fall neutral verlaufender Spannuten.

Ganz anders stellt sich die Situation für schräg verlaufende Spannuten dar, denn hier ist die Schneidkraft je nach Drallrichtung auf eine der beiden Flanken des Gewindezahns verlagert. Der Grad der Verlagerung hängt dabei von dem Drallwinkel der Spannut ab. Bei größeren Drallwinkeln liegt dann die Schneidkraft total einseitig. Damit nun bei dem Einschraubvorgang einer derartigen Schraubpfanne tatsächlich die einseitig vorhandene Schneidkante in der vorgesehenen Weise ihre Wirkung entfalten kann, ist es erforderlich das im wesentlichen in radialer Richtung stattfindende "Schieben" des Zahnprofils richtungsmäßig zu beeinflussen. Erfindungsgemäß geschieht dieses durch die Heranziehung des oben bereits erwähnten Korrekturwinkels bei der Festlegung der korrigierten Projektionslinien derart, daß diese um ihren Drehpunkt von der mit Schneidkraft stärker belasteten Zahnflanke weggedreht werden. Als vorteilhaft wird diesbezüglich vorgeschlagen, den Grad der Korrektur proportional dem Verhältnis der jeweiligen flankenseitigen Gesamtschnittkräfte, also der Summe der Schneid-, Raspel- und Verdrängungskräfte vorzunehmen.

Für die grobe Bestimmung der Variation der Gewindesteigung müssen von der zu fertigenden Schraubpfanne der Kippwinkel des Gewindezahnprofils, der Drallwinkel der Spannuten, und die Oberflächenbeschaffenheit bekannt sein. Aufgrund dieser Daten ist eine überschlägige Festlegung der auf den Gewindezahn beim Einschrauben einwirkenden Kraftverteilung möglich. Für die Erzielung eines absoluten Minimums des Einschraubkraftbedarfs wird die Durchführung von Einschraubversuchen anhand von Prototypen empfohlen.

Diese Vorgehensweise soll im folgenden anhand eines Beispiels erklärt werden. Nimmt man an, daß das Versuchsmuster einer konventionellen Schraubpfanne des Nenndurchmessers 54 mm mit einem Spitzgewinde (Zahnhöhe 2,5 mm, Gewindesteigung 4 mm, Gewindeerstreckung 16 mm) im gedrehten Zustand, also mit relativ glatter Oberfläche, beim Einschrauben in einen Hartschaumkörper bis zum Aufsetzen des Schalenmantels ein maximales Drehmoment von 20 Nm erfordert, so kann mit einem zweiten Versuch das entsprechende Einschraubdrehmoment für eine gesandstrahlte Version (Rauhigkeit $R_a$ z.B. 4 $\mu$m) z.B. mit 50 Nm ermittelt werden. Der Anteil der Raspelkräfte am Gesamt-Einschraubdrehmoment beträgt mit 30 Nm dann genau 60%. Diese Raspelkräfte sind über die Oberfläche gleichmäßig verteilt, betragen also demgemäß 30% pro Gewindezahnflanke. Demgegenüber ist das zuvor ermittelte Grund-Drehmoment in Höhe von 20 Nm (entsprechend 40%) im wesentlichen durch Schnittkräfte mit einem gewissen Anteil an Quetsch- bzw. Verdrängungskräften begründet. Wenn diese Schnittkräfte bei einem Drall der Spannut total einseitig auf der Flanke des Gewindezahns liegen, beträgt der Anteil am gesamten Einschraubdrehmoment auf der mit der Schneidkante versehenen Flanke des Gewindezahns 70%, sodaß 30% für die abgewandte Seite verbleiben. Beträgt nun der Kippwinkel der Mittellinie des Gewindezahns 10° und der eingeschlossene Flankenwinkel 20°, dann wird aus der Mittellinie die korrigierte Projektionslinie abgeleitet, indem die Einschraubkraftverteilung mit 70% zu 30% zum Flankenwinkel ins Verhältnis gesetzt wird. Bei einem Rechtsgewinde mit Rechtsdrall der Spannuten errechnet sich der Kippwinkel der Projektionslinien daraus zu 14°.

Nach dem Festlegen der Linienschar korrigierter Projektionslinien auf der Grundlage der angestrebten mittleren Gewindesteigung, des Kippwinkels des Zahnprofils und der angestrebten Schnittkraftverteilung besteht der nächste Schritt darin, auf der gewünschten Schalengeometrie der Schraubpfanne (z.B. sphärisch, parabolisch, usw.) die jeweilige Zahnhöhe der einzelnen Umläufe der Gewinderippe und jeweils den oben genannten Projektionspunkt festzulegen. Von diesem ausgehend werden dann die gewählten Seitenwinkel des Gewindezahnprofils aufgetragen und ihr jeweiliger Fußpunkt auf dem Schalenmantel ermittelt. Diese Fußpunkte sind die Basis für die Erstellung eines Bearbeitungsprogramms für die Herstellung des erfindungsgemäßen Gewindes auf einer numerisch gesteuerten Maschine.

Generell sind verschiedene Verfahren für die Herstellung von speziellen Gewinden aus der Patentliteratur bekannt, welche teilweise so weit angepaßt oder abgeändert werden können, daß sie für die Herstellung des hiermit zur Verfügung gestellten Spezialgewindes und der entsprechenden Implantate nutzbar sind. Insbesondere mit der Deutschen Patentanmeldung P 44 00 001.4 und der daraus hervorgegangenen Internationalen Anmeldung WO 95/18586 werden verschiedene Möglichkeiten zur Bearbeitung eines Gewindes mit veränderlich modifizierbarem Gewindeprofil angeboten. Diese bestehen in der Benutzung unterschiedlicher Steigungen, auch mit einem sich kontinuierlich ändernden Wert der Steigung, und der Heranziehung verschiedener Offset-Werte für das oder die Bearbeitungswerkzeuge, wobei diese Parameter in der zitierten Schrift lediglich für die Beeinflussung des Gewindeprofils herangezogen werden und dabei die eigentliche Gewindesteigung in axialer Richtung konstant bleibt. Unter Benutzung dieses Verfahrens ist es jedoch auch möglich, im Bearbeitungszyklus in schräg stehende Gewinderillen einzutauchen, den Gewindegrund mit höchster Auflösung an die Sollkontur anzupassen, und gleichzeitig nicht nur die Zahnhöhe, sondern auch die jeweilige Zahnposition gemäß der mit der vorliegenden Erfindung gegebenen Lehre genauestens zu verwirklichen. Besonders kostengünstig ist die spanende Herstellung auf einer CNC-Drehmaschine. Bei modernen Steuerungen solcher Maschinen ist z.B. mit dem Befehlssatz G 33 unter E die kontinuierliche Änderung der Gewindesteigung direkt programmierbar. Bestimmte Funktionen der Steigungsänderung sind z.B. durch die Verkettung von derartigen Befehlssätzen bzw. die Einfügung entsprechender Unterprogramme (z.B. mit einer Variablenprogrammierung) zu erreichen. Für den Durchschnittsfachmann dürfte es keine besonderen Schwierigkeiten bereiten, solche CNC-Programme maschinenspezifisch zu erstellen.

Die Erfindung soll im folgenden anhand der fünf Zeichnungsfiguren näher erläutert werden. **Fig**.1 zeigt ein Schema zur konstruktiven Festlegung des Gewinderippenzuges an einem Beispiel, **Fig**.2 die winkelmäßigen Definitionen an einem vergrößert gezeichneten Gewindezahn aus dem Beispiel gemäß **Fig**.1, und **Fig**.3 das Beispiel einer erfindungsgemäßen Hüftgelenkpfanne. In **Fig**.4 ist ein konventioneller Gewindeanfang einer Hüftgelenkpfanne und in **Fig**.5 ein Gewindeanfang gemäß der Erfindung schematisch dargestellt.

**Fig**.1 soll die Vorgehensweise zur Festlegung des erfindungsgemäßen Spezialgewindes anhand eines beispielhaften Schemas verdeutlichen. Es ist ein Konturzug **1** als Teilstück des Umrisses eines halbseitigen Axialschnittes einer Schraubpfanne dargestellt, wobei für das Beispiel eine sphärische Mantelform gewählt wurde. Der umlaufende Gewindezug ist im Schnitt durch die einzelnen Zähne **A, B, C, D** und **E** repräsentiert, welche in dieser Reihenfolge vom äquatornahen Rand der Pfanne zur polseitigen Kuppel hin auf dem sphärischen Schalenmantel verteilt sind. Die Gewindezähne sind in ihrem Profil als spitzwinklige Dreiecke ausgeführt und schließen mit ihren Flanken einen Winkel von 22° ein. Sie sind ferner um 21° aus der neutralen Mittellage heraus in Richtung zum Pfannenpol gekippt. Eine Schar von geraden, parallel und jeweils mit dem gleichen Abstand **a** zueinander aufgetragenen Linien **2, 3, 4, 5** und **6**, die sogenannten korrigierten Projektionslinien, wurden benutzt, um die Positionen der einzelnen Gewindezähne festzulegen. Die Projektionslinien verlaufen innerhalb des von den Flanken der Gewindezähne eingeschlossenen Winkels, wobei ihr Kippwinkel hier mit 30° angenommen wurde, weil das Beispiel für stark rechtsgängig gedrallte Spannuten gelten soll. Die Spannuten selbst sind in der Darstellung nicht sichtbar. Sie bewirken wegen ihres stärkeren Rechtsdralls eine Ausbildung von Schneidkanten auf den in der Zeichnung rechts, also polseitig liegenden Flanken der Gewindezähne. Deshalb ist zur Erzielung einer bestimmungsgemäßen Funktion dieser Schneidkanten eine stärkere Verlagerung der Schnittkräfte auf die polseitige Flanke anzustreben. Dieses wird erfindungsgemäß durch das winkelmäßige Wegdrehen der Projektionslinien um den Korrekturwert bewirkt, woraus sich beim Einschrauben ein entsprechendes "Schieben" des Zahnquerschnitts in eine bestimmte Wirkrichtung ergibt. Für das herangezogene Beispiel wurde für die Oberfläche der Schraubpfanne eine z.B. durch Sandstrahlen hergestellte feine Aufrauhung angenommen. Weil daraus beim Einschrauben zu beiden Seiten des Gewindezahns ein gewisser Anteil an Raspelkräften resultiert, wurde die Schnittkraftverteilung zwischen den Zahnflanken auf 9 zu 91 festgelegt. In dem Beispiel sollen demgemäß 91% der Schnittkraft von der polseitigen, und 9% von der äquatorseitigen Gewindezahnflanke erbracht werden. Daher ist die korrigierte Projektionslinie in der Zeichnungsfigur nur mit 30° Kippwinkel zur Senkrechten angesetzt, während der entsprechende Flankenwinkel des Gewindezahns mit 32° um 2° größer ist. Als Drehpunkt der Projektionslinien dient das jeweils eingezeichnete Mittelpunktskreuz, welches in vorliegenden Fall mit den jeweiligen Gewindezahnspitzen übereinfällt und sich aus der konstruktiv gewählten Zahnhöhe ergibt. Die Mittelpunktskreuze an den Zahnspitzen wurden mit den senkrecht zur Pfannenachse stehenden Konstruktionshilfslinien **7, 8, 9, 10** und **11** gebildet, welche anhand ihrer axial gemessenen Abstände **b, c, d** und **e** die an den Zahnspitzen gemessene Änderung der Gewindesteigung aufzeigen. Zum Vergleich ist die originäre Steigung **s** als horizontaler Abstand der Projektionslinien aufgetragen. Es soll noch darauf hingewiesen werden, daß für die Erstellung eines geeigneten CNC-Bearbeitungsprogramms die Gewindesteigung im Bereich der Fußpunkte der Gewindezähne synchron mit dem entsprechenden Bezugspunkt der Werkzeugbeschreibung verwendet werden muß.

Zum besseren Verständnis der in **Fig**.1 gezeigten Situation und zur übersichtlicheren Darstellung der verschiedenen Winkel wurde in der **Fig**.2 der Gewindezahn **C** vergößert herausgezeichnet. Der Konturzug **1** ist entsprechend als Teilabschnitt dargestellt. Die polseitige Flanke **12** des Gewindezahnes ist mit dem Winkel α von 10° rechtsdrehend von der senkrechten Konstruktionshilfslinie **9** weggeschwenkt. Der entsprechende Seitenwinkel β der Zahnflanke 13 beträgt 32°. Es ergibt sich so ein zwischen den Zahnflanken eingeschlossener Winkel δ von 22°. Die strich-gepunktet gezeichnete Winkelhalbierende **15** ist gleichzeitig die unkorrigierte Projektionslinie, welche um den Drehpunkt **14** (hier gleichzeitig Gewindezahnspitze) drehbar ist. Ihr Kippwinkel ist mit γ bezeichnet. Die Projektionslinie **15** wurde mit einem Winkel von 9° rechtsschwenkend korrigiert und so die korrigierte Projektionslinie **4** mit dem Schwenkwinkel χ von 30° gebildet.

Das Beispiel einer praktischen Ausführung der erfindungsgemäßen Hüftgelenkpfanne wird in **Fig**.3 gezeigt. Dabei wurde die Gewindegestaltung an das Schema aus **Fig**.1 bzw. **Fig**.2 angelehnt. Die vorgestellte hemisphärische Hüftgelenkpfanne **16** ist teilweise aufgebrochen und geschnitten dargestellt, um den Verlauf der Wandung und des Gewindes besser verdeutlichen zu können. Die innere Gestaltung der Schraubpfanne ist in einen zylindrischen Bereich **17** und einen Hohlkugelabschnitt **18** für die Aufnahme eines nicht dargestellten Inletts untergliedert. Die Pfannenwandung ist im äquatornahen Bereich **19** dicker gewählt, um Nuten für das erforderliche Einschraubwerkzeug einfräsen zu können. Zur Pfannenkuppel **20** hin nimmt die Wandstärke ab. Es schließt sich ein Bodenloch **21** an, welches eine Sichtkontrolle des Pfannensitzes beim Implantieren ermöglicht. Das gezeigte Gewinde ist eingängig ausgeführt. Im Schnitt ist die Verteilung der Zähne **22, 23, 24, 25** und **26** des Gewindezuges auf dem Schalenmantel zu erkennen. Die Zähne sind deutlich sichtbar in Richtung zum Pfannenpol gekippt. Im Gewindezug sind durch entsprechende Schlitzung mit einem Radiusfräser Spannuten gebildet, von denen drei (**27, 28** und **29**) sichtbar sind. Die Spannuten sind rechtsgängig mit einem Winkel φ zur Pfannenachse gedrallt, um auf der polseitigen Flanke Schneidkanten mit einem positiven Spanwinkel zu bilden.

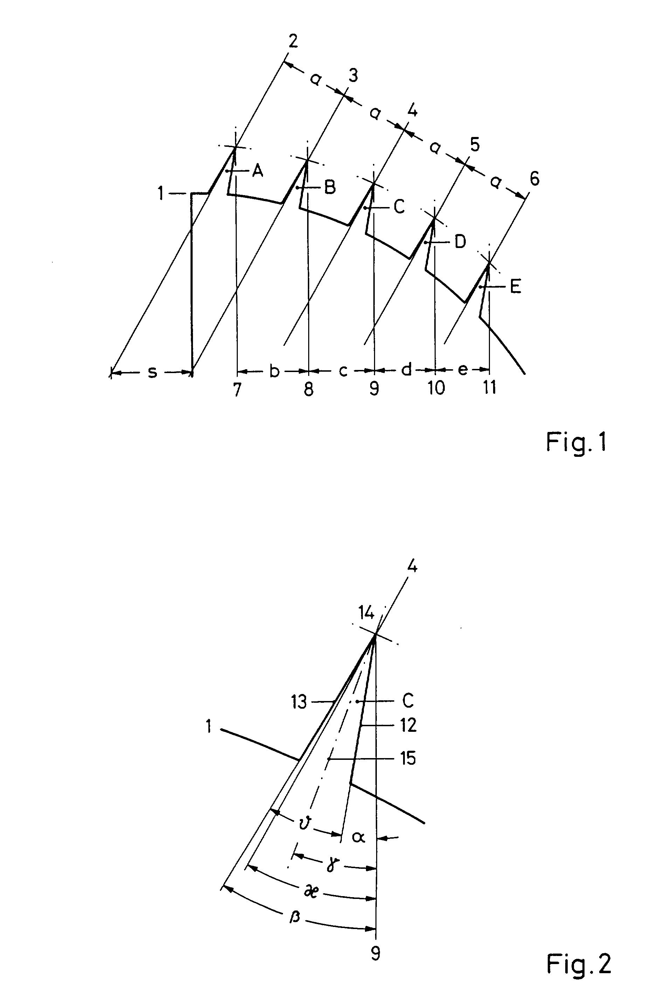

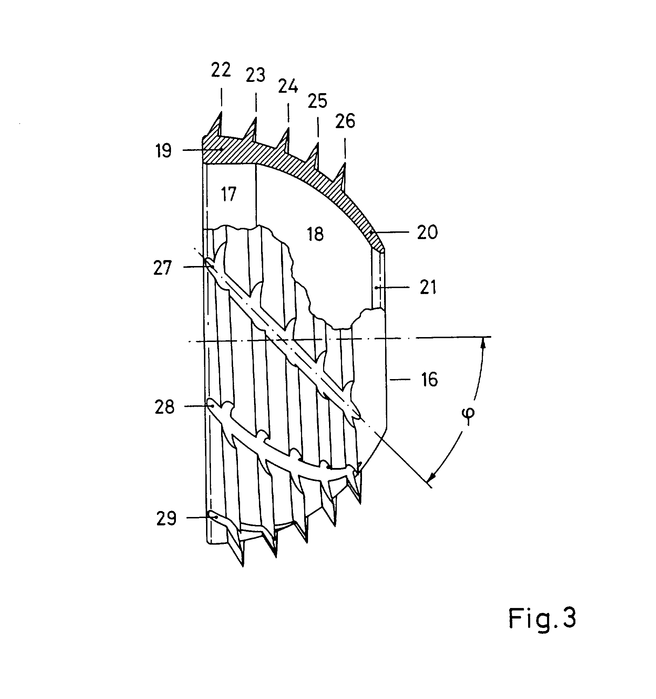

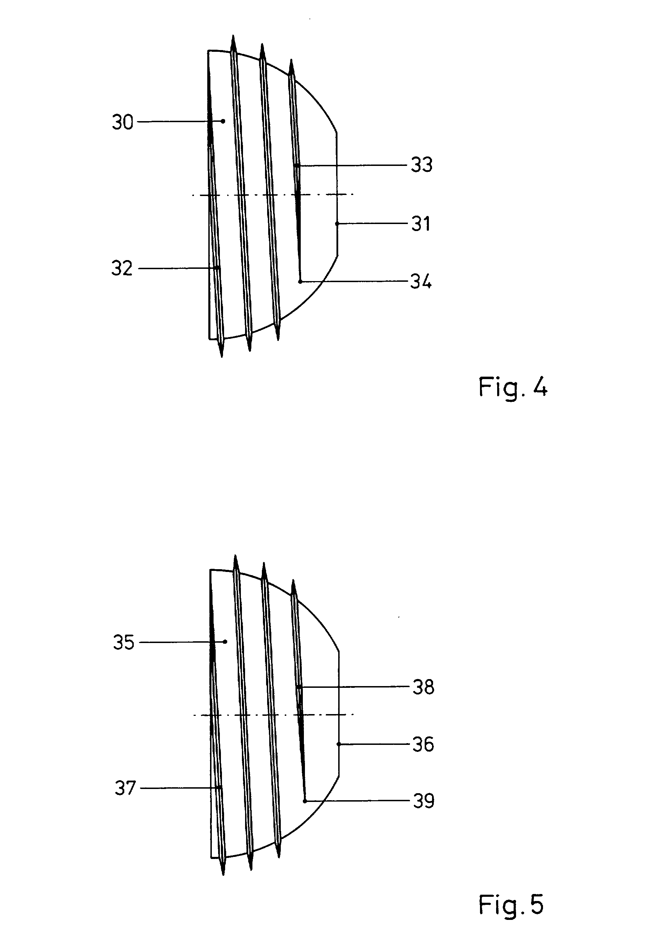

In der zeichnerisch vereinfachten **Figur** 4 ist eine herkömmliche Schraubpfanne mit Spitzgewinde dargestellt, wobei z.B. perspektivische Verzerrungen des Gewindes nicht berücksichtigt wurden. Die Schraubpfanne (**30**) besitzt einen hemisphärischen Schalenmantel. Wegen des Bodenlochs entsteht in der Seitenansicht eine Abplattung (**31**). Das Gewindezahnprofil steht neutral, also ohne Kippwinkel. Die Spannuten wurden aus Gründen der Vereinfachung weggelassen. Vom Gewindeanfang (**34**) wächst der Gewindezahn bis zum Übergangspunkt (**33**) auf seine volle Höhe an. Dann läuft die Gewinderippe (**32**) bis zum Pfannenäquator etwa dreimal um. Die zum Pfannenpol zeigende Flanke des Gewindezahns weist zwischen den Punkten **34** und **33** keine Steigung auf. Sie ist das Relikt eines Kegelrings, welcher aus der üblichen drehtechnischen Vorbearbeitung resultiert. Durch diese relativ zum Verlauf des übrigen Gewindezugs merkliche Schiefstellung dieses Flankenbereichs sind die beim Eindrehen der Schraubpfanne auftretenden Kräfte ungleichmäßig auf die Flanken verteilt. Dadurch entsteht beim Einschrauben bzw. beim eventuellen Vorschneiden mit einem Werkzeug ein unnötig hoher Kraftbedarf.

Zum Vergleich wird in **Fig**.5 das Beispiel einer Schraubpfanne mit erfindungsgemäßem Gewindeanfang gezeigt. Wegen der Vergleichbarkeit wurde die Schraubpfanne (**35**) in den übrigen Einzelheiten unverändert übernommen, sodaß ihre hemisphärische Formgestalt, die Abplattung (**36**), und die Gewinderippe zwischen den Punkten **38** und **37** der in **Fig**.4 gezeigten Schraubpfanne entsprechen. Die Strecke vom Gewindeanfang (**39**) bis zum Übergangspunkt (**38**) wurde durch Verlagern des Gewindeanfangs in Richtung zum Pfannenpol hin so verschwenkt, daß die auf die Gewindeflanken wirkenden Kräfte ausgeglichen sind. Dabei wurde die polseitige Zahnflanke mit einer kleineren, bzw. die äquatorseitige Zahnflanke mit einer größeren als die übrige Steigung geschnitten. Mit dieser Gestaltung sind die Einschraubkräfte beim Implantieren der Schraubpfanne, bzw. die Schneidkräfte beim Vorschneiden gegenüber bisherigen Ausführungen merklich reduziert.

Mit der Erfindung wird insgesamt eine Schraubpfanne mit einer Variationsbreite zur Verfügung gestellt, welche die Anwendung von ein- oder mehrgängigem Rechts- oder Linksgewinde, neutralen oder rechts- bzw. linksgedrallten Spannuten und nahezu beliebigen Kippwinkeln des Gewindezahnprofils bei sämtlichen möglichen Formen des Schalenmantels erlaubt. Wegen der Anpaßbarkeit der Steigungsvariation im Gewindezug an die jeweilige Bauform und die gezielte Verteilbarkeit der Einschraubkräfte auf die Gewinderippe ist für jede Ausführung das kleinstmögliche Einschraubdrehmoment erzielbar, auch bei unterschiedlichen Oberflächenrauhigkeiten. Dabei wird nicht nur ein frühzeitiges Verklemmen der Schraubpfanne beim Eindrehen, bzw. die Gefahr der Beckensprengung vermieden, sondern auch insbesondere die Position des Aufsetzens des Schalenmantels auf das knöcherne Lager besser fühlbar. Bei der besonders empfohlenen Kippung der Gewindezähne in Richtung zum Pfannenpol wird in Verbindung mit der nichtlinearen Gewindesteigung eine merkliche Reduzierung der Lastspitzen beim bestimmungsgemäßen Gebrauch des Implantats erreicht. Die erfindungsgemäße Schraubpfanne ist daneben problemlos und ohne zusätzliche Kosten herstellbar, z.B. auf einer CNC-Drehmaschine. Damit versprechen die Eigenschaften der erfindungsgemäßen Schraubpfanne einen Fortschritt in diesem Teilbereich der Medizintechnik.

**Patentansprüche**

**1.** Einschraubbare künstliche Hüftgelenkpfanne mit einer im Gewindebereich liegenden, stetig oder stufig aufgelösten Krümmung der äußeren Axialkontur ihres Schalenkörpers, mit einem selbstschneidenden Gewinde zum Einschrauben in das Acetabulum, mit gedrallten Spannuten und/oder einem aus der Radialebene gekippten Gewindezahnprofil, <u>dadurch gekennzeichnet</u>, daß die Gewindesteigung in axialer Richtung variiert und dabei durch die folgenden Parameter bestimmt wird:

a) Krümmung der äußeren Axialkontur des Schalenkörpers
b) Kippwinkel des Gewindezahnprofils
c) Drallwinkel der Spannut und
d) Rauhigkeit der Gewindeoberfläche.

**2.** Einschraubbare Hüftgelenkpfanne nach Anspruch 1, deren Gewinde im polseitigen Anfangsbereich zwischen dem Auftauchen (**39**) des Gewindezahns und dem Erreichen seiner vollen Zahnhöhe (**38**) eine Steigung der polseitigen Gewindezahnflanke aufweist, die kleiner, und eine Steigung der äquatorseitigen Gewindezahnflanke, die größer ist, als die restliche Steigung des Schraubpfannengewindes.

**3.** Einschraubbare künstliche Hüftgelenkpfanne nach Anspruch 1 oder 2, deren Gewindezahnprofil mit einem Winkel ( γ ) aus der Radialebene gekippt ist und deren Gewinde entweder keine Spannuten besitzt oder wenn Spannuten vorhanden sind, diese einen Spannutendrallwinkel von Null aufweisen, wobei die in einem entsprechenden halbseitigen Axialschnitt der Hüftgelenkpfanne verteilten Gewindezähne des Gewindes mit ihren jeweiligen Mittellinien (**15**) auf Äquidistanten, also parallel und jeweils im gleichen Abstand zueinander verlaufenden Linien, angeordnet sind.

4. Einschraubbare künstliche Hüftgelenkpfanne nach einem der vorhergehenden Ansprüche, wobei das selbstschneidende Gewinde mit außen am Zahnkopf liegenden, radial wirkenden Schneidkanten wie bei üblichen Flachgewinden zur zementfreien Verankerung im Acetabulum versehen und der auf dem Schalenmantel gebildete Gewinderippenzug mindestens partiell strukturiert oder aufgerauht ist, und zwecks Kompensierung der Raspelwirkung der Gewindezahnflanken diese zum Zahnkopf hin mit einem Winkel zwischen 0,5 und 5° aufeinander zu verlaufen.

5. Einschraubbare künstliche Hüftgelenkpfanne nach Anspruch 1 oder 2, mit einem sich zum Gewindezahnkopf hin mit beliebigem Winkel verjüngenden Gewindezahnprofil und gedrallten Spannuten (**27, 28, 29**), deren im halbseitigen Axialschnitt der Hüftgelenkpfanne auf dem Schalenmantel verteilten Gewindezähne **A, B, C, D, E** des Gewindes mit dem Schnittpunkt (**14**) ihrer an die Gewindezahnflanken (**12, 13**) gelegten Seitenlinien auf korrigierte Projektionslinien genannten Äquidistanten (**2, 3, 4, 5, 6**), also parallel und jeweils im gleichen Abstand zueinander verlaufenden Linien angeordnet sind, deren winkelmäßige Lage innerhalb der beiden Gewindezahnflanken (**12, 13**) festgelegt und deren Drehpunkt der Schnittpunkt (**14**) der an die Gewindezahnflanken gelegten Seitenlinien ist.

6. Einschraubbare künstliche Hüftgelenkpfanne nach Anspruch 1 oder 2, deren Gewinde ein sich zum Gewindezahnkopf hin mit beliebigem Winkel verjüngendes, mit einem Winkel ( γ ) aus der Radialebene gekipptes Gewindezahnprofil und gedrallte Spannuten (**27, 28, 29**) besitzt, wobei die im halbseitigen Axialschnitt der Hüftgelenkpfanne auf dem Schalenmantel verteilten Gewindezähne (**22, 23, 24, 25, 26**) mit dem Schnittpunkt (**14**) ihrer an die Gewindezahnflanken (**12, 13**) gelegten Seitenlinien auf korrigierte Projektionslinien genannten Äquidistanten (**2, 3, 4, 5, 6**), also parallel und jeweils im gleichen Abstand zueinander verlaufenden Linien angeordnet sind, deren winkelmäßige Lage innerhalb der beiden Gewindezahnflanken (**12, 13**) festgelegt und deren Drehpunkt der Schnittpunkt (**14**) der an die Gewindezahnflanken gelegten Seitenlinien ist.

7. Einschraubbare künstliche Hüftgelenkpfanne nach Anspruch 1, deren Gewinde ein mit einem Winkel ( γ ) aus der Radialebene gekipptes Gewindezahnprofil mit parallelen Flanken und gedrallte Spannuten besitzt, wobei die im halbseitigen Axialschnitt der Hüftgelenkpfanne auf dem Schalenmantel verteilten Gewindezähne auf korrigierte Projektionslinien genannten Äquidistanten (**2, 3, 4, 5, 6**), also parallel und jeweils im gleichen Abstand zueinander verlaufenden Linien angeordnet sind, deren winkelmäßige Lage innerhalb der beiden Gewindezahnflanken festgelegt und deren Drehpunkt die Mitte der Stirnfläche des Gewindezahnkopfes ist.

8. Einschraubbare künstliche Hüftgelenkpfanne nach Anspruch 5, 6 oder 7, deren korrigierte Projektionslinien von der Mittellinie (**15**) in Richtung zur schnittkraftmäßig weniger zu belastenden Flanke des Gewindezahnprofils verschwenkt sind.

9. Einschraubbare künstliche Hüftgelenkpfanne nach Anspruch 8, mit korrigierten Projektionslinien, deren Grad der winkelmäßigen Verschwenkung dem Verhältnis der an den Gewindezahnflanken zu bewirkenden Schnittkräfte proportional ist, wobei der Winkel $\chi$ der korrigierten Projektionslinien (**2, 3, 4, 5, 6**) im wesentlichen beschreibbar ist durch die Beziehung:

$$\text{Winkel } \chi = \text{Winkel } \alpha + \frac{\text{Winkel } \delta \cdot \text{Kraft } F_{PA} \, (\%)}{100},$$

wobei

$\chi$ = Winkel der korrigierten Projektionslinien (in Grad)
$\alpha$ = polseitiger Gewindezahnseitenwinkel (in Grad)
$\delta$ = eingeschlossener Gewindezahnflankenwinkel (in Grad)
**$F_{PA}$** = angestrebter prozentualer polseitiger Anteil von den an den beiden Gewindezahnflanken insgesamt wirkenden Komplexkräften aus Schneid-, Raspel- und Quetsch- bzw. Verdrängungskräften.

10. Einschraubbare künstliche Hüftgelenkpfanne gemäß einem oder mehreren der vorhergehenden Ansprüche, mit einem in Richtung zum Pfannenpol hin gekippten Gewindezahnprofil, wobei die polseitige Flanke (**12**) des Gewindezahns (**C**) mit einem Winkel ( α ) über die Radialebene hinaus in Richtung zum Pfannenpol hin geneigt ist.

**Claims**

1.  Screw-in type artifial hip joint socket, exhibiting within its threaded portion a progressively or stepwise resolved curvature of the shell body exterior axial contour, the socket being provided with a self-tapping thread for screwing into the acetabulum and with at least one of twisted tapping grooves and/or a thread tooth profile tilted away from the radial plane, thereby characterized, that the thread pitch varies in the axial direction depending upon the following parameters:

    a) the curvature of the shell body exterior axial contour,
    b) the angle of tilt of the thread tooth profile,
    c) the twist angle of the tapping groove,
    d) the roughness of the thread surface.

2.  Screw-in type artificial hip joint socket according to claim 1, wherein the thread in the pole side starting portion between the initial surfacing (**39**) of the thread tooth and the achievement of a full tooth height (**38**) is provided with a pitch of the pole side thread tooth flank which is smaller, and a pitch of the equator side thread tooth flank which is larger than the residual pitch of the socket thread.

3.  Screw-in type artificial hip joint socket according to claim 1 or 2, whose thread tooth profile is tilted away at an angle (γ) from the radial plane, and either no tapping grooves are provided within the thread or, if tapping grooves are provided, these run with a twist angle of zero, wherein the thread teeth of the thread distributed in a corresponding semilateral axial section of the hip joint socket are placed with their respective bisection lines (**15**) on equidistants, i.e. lines that run parallel and at the same distance to each other.

4.  Screw-in type artificial hip joint socket according to one of the preceding claims, whereby the self-tapping thread is provided with radially effective cutting edges outlying on the tooth head as in the conventional flat threads for cementless anchoring in the acetabulum, the thread ridge procession formed upon the shell mantle at least being partially structured or roughened, and in order to compensate the rasping effect of the thread tooth flanks these run in direction to the tooth head with an enclosed angle between 0,5 and 5° towards each other.

5.  Screw-in type artificial hip joint socket according to claim 1 or 2, wherein the thread tooth profile is narrowing towards the thread tooth head at any angle, and with twisted tapping grooves (**27, 28, 29**), wherein the thread teeth **A, B, C, D, E** of the thread distributed upon the shell mantle in the semilateral axial section of the hip joint socket are placed with the point of intersection (**14**) of their side lines joining the thread tooth flanks (**12, 13**) upon equidistants (**2, 3, 4, 5, 6**) designated as corrected projection lines, thus parallel and respectively with the same distance to each other running lines, of which the angular position is determined between the thread tooth flanks (**12, 13**), using the point of intersection (**14**) of the side lines joining the thread tooth flanks as the angular rotation point.

6.  Screw-in type artificial hip joint socket according to claim 1 or 2, wherein the thread exhibits a thread tooth profile narrowing towards the thread tooth head at any angle, being tilted with an angle (γ) away from the radial plane and provided with twisted tapping grooves (**27, 28, 29**), wherein the thread teeth (**22, 23, 24, 25, 26**) distributed upon the shell mantle in a semilateral axial section of the hip joint socket are placed with the point of intersection (**14**) of their side lines joining the tooth flanks (**12, 13**) upon equidistants (**2, 3, 4, 5, 6**) designated as corrected projection lines, thus parallel and respectively with the same distance to each other running lines, of which the angular position is determined between the two flanks (**12, 13**) of the thread tooth, wherein the point of intersection (**14**) of their joining side lines is used as the angular rotation point.

7.  Screw-in type artificial hip joint socket according to claim 1, wherein the thread exhibits a thread tooth profile with parallel flanks, being tilted with an angle (γ) away from the radial plane and provided with twisted tapping grooves, wherein the thread teeth distributed upon the shell mantle in a semilateral axial section of the hip joint socket are placed upon equidistants (**2, 3, 4, 5, 6**) designated as corrected projection lines, thus parallel and respectively with the same distance to each other running lines, of which the angular position is determined between the two thread tooth flanks, wherein the center of the thread tooth head is used as the angular rotation point.

8.  Screw-in type artificial hip joint socket according to claim 5, 6 or 7, wherein the corrected projection lines are slewed away from the bisection line (**15**) in the direction towards the thread tooth flank to be less burdened with cutting forces.

9.  Screw-in type artificial hip joint socket according to claim 8, wherein the degree of the angular rotation of the corrected projection lines is proportional to the relationship of the cutting forces to be acting on the thread tooth flanks, the angle χ of the corrected projection lines (**2, 3, 4, 5, 6**) being described essentially by the equation:

$$\text{Angle } \chi = \text{Angle } \alpha + \frac{\text{Angle } \delta \cdot \text{Force } F_{PA} \ (\%)}{100},$$

where

$\chi$ = angle of the corrected projection lines (in degrees)

$\alpha$ = pole side thread tooth side angle (in degrees)

$\delta$ = enclosed thread tooth angle (in degrees)

$F_{PA}$ = desired percentile pole side portion of the totally acting complex forces upon both thread tooth flanks, composed of cutting-, rasping-, and compressing- or, as the case may be displacement forces.

10. Screw-in type artificial hip joint socket according to one or several of the preceding claims, wherein the thread tooth profile is tilted in the direction to the socket pole in slewing the pole side flank (**12**) of the thread tooth (**C**) with an angle ($\alpha$) beyond the radial plane in direction to the socket pole.

## Revendications

1. Cavité cotyloïde artificielle vissable comportant une courbure, réduite constamment ou par paliers se situant dans la zone de filet, du contour axial externe de son corps de coque, comprenant un filet autotaraudeur pour le vissage dans l'acétabule, avec des rainures à copeaux hélicoïdaux et/ou un profil de dent de filetage basculé à partir du plan radial, caractérisée en ce que le pas de filetage varie dans le sens axial tout en étant déterminé par les paramètres suivants :

   a) courbure du contour axial externe du corps de coque
   b) angle de basculement du profil de dent de filetage
   c) angle de torsion de la rainure à copeaux et
   d) rugosité de la surface de filet.

2. Cavité cotyloïde vissable selon la revendication 1 dont le filet dans la zone initiale côté pôle présente, entre l'émergence (**39**) de la dent de filetage et l'atteinte de sa hauteur de dent complète (**38**), un pas du flanc de la dent de filetage côté pôle qui est plus petit, et un pas du flanc de la dent côté équateur qui est plus grand que le pas restant du filet de la cavité cotyloïde vissable.

3. Cavité cotyloïde artificielle vissable selon la revendication 1 ou 2 dont le profil de dent de filetage est basculé avec un angle ($\gamma$) à partir du plan radial et dont le filet soit ne possède aucune rainure à copeaux, ou si des rainures à copeaux existent, celles-ci présentent un angle de torsion de rainure à copeaux de zéro, les dents de filetage de filet réparties dans une coupe axiale partielle correspondante de la cavité cotyloïde étant disposées avec leurs lignes médianes respectives (**15**) sur des équidistantes, donc sur des lignes courant parallèlement et respectivement à la même distance les unes par rapport aux autres.

4. Cavité cotyloïde artificielle vissable selon l'une des revendications précédentes dans laquelle le filet autotaraudeur est pourvu d'arêtes de coupe agissant radialement et se situant à l'extérieur sur la tête de dent comme pour les filets usuels destinés à l'ancrage sans ciment dans l'acétabule, et le train nervuré de filetage formé sur le manteau de coque est au moins partiellement structuré ou grenelé, et, dans le but de compenser l'effet de râpe des flancs de dent de filetage, ceux-ci se rapprochent vers la tête de la dent avec un angle compris entre 0,5 et 5°.

5. Cavité cotyloïde artificielle vissable selon la revendication 1 ou 2 comprenant un profil de dent de filetage s'amincissant vers la tête de dent de filetage avec un angle quelconque et des rainures à copeaux hélicoïdaux (**27, 28, 29**) dont les dents de filetage **A, B, C, D, E** du filet réparties dans la coupe axiale partielle de la cavité cotyloïde sur le manteau de coque sont disposées avec le point de coupe (**14**) de leurs lignes latérales situées sur les flancs de dent de filetage (**12, 13**) sur des équidistantes (**2, 3, 4, 5, 6**) nommées lignes de projection corrigées, et donc sur des lignes courant parallèlement et respectivement à la même distance les unes par rapport aux autres dont la position angulaire est fixée à l'intérieur des deux flancs de dent de filetage (**12, 13**) et dont le point de rotation est le point de coupe (**14**) des lignes latérales situées sur les flancs de dent de filetage.

6. Cavité cotyloïde artificielle vissable selon la revendication 1 ou 2 dont le filet possède un profil de dent de filetage s'amincissant vers la tête de dent de filetage avec un angle quelconque et basculé avec un angle ($\gamma$) à partir du plan radial et des rainures à copeaux hélicoïdaux (**27, 28, 29**), les dents de filetage (**22, 23, 24, 25, 26**) réparties dans la coupe axiale partielle de la cavité cotyloïde sur le manteau de coque étant disposées avec le point de coupe (**14**) de leurs lignes latérales situées sur les flancs de dent de filetage (**12, 13**) sur des équidistantes (**2, 3, 4, 5, 6**) nommées lignes de projection corrigées, et donc sur des lignes courant parallèlement et respectivement à la même distance les unes par rapport aux autres dont la position angulaire est fixée à l'intérieur des deux flancs de dent de filetage (**12, 13**) et dont le point de rotation est le point de coupe (**14**) des lignes latérales situées sur les flancs de dent de filetage.

7. Cavité cotyloïde artificielle vissable selon la revendication 1 dont le filet possède un profil de dent de filetage, basculé avec un angle (γ) à partir du plan radial, avec des flancs parallèles et des rainures à copeaux hélicoïdaux, les dents de filetage réparties dans la coupe axiale partielle de la cavité cotyloïde sur le manteau de coque étant disposées sur des équidistantes (**2, 3, 4, 5, 6**) nommées lignes de projection corrigées, et donc des lignes courant parallèlement et respectivement à la même distance les unes par rapport aux autres dont la position angulaire est fixée à l'intérieur des deux flancs de dent de filetage et dont le point de rotation est le milieu de la face de la tête de la dent de filetage.

8. Cavité cotyloïde artificielle vissable selon la revendication 5, 6 ou 7 dont les lignes de projection corrigées sont pivotées à partir de la ligne médiane (**15**) en direction du flanc du profil de dent de filetage susceptible d'être moins sollicité en matière de force de coupe.

9. Cavité cotyloïde artificielle vissable selon la revendication 8, avec des lignes de projection corrigées dont le degré de pivotement angulaire est proportionnel au rapport des forces de coupe à exercer sur les flancs de dent de filetage, l'angle χ des lignes de projection corrigées (**2, 3, 4, 5, 6**) étant descriptible essentiellement par la relation:

$$\text{angle } \chi = \text{angle } \alpha + \frac{\text{angle } \delta \cdot \text{force } F_{PA} \text{ (\%)}}{100},$$

où

χ = angle des lignes de projection corrigées (en degré)
α = angle azimutal de dent de filetage côté pôle (en degré)
δ = angle de flanc de dent de filetage inclus (en degré)
**F$_{PA}$** = part en pourcentage côté pôle désirée des forces complexes agissant au total sur les deux flancs de dent de filetage à partir des forces de coupe, râpe et d' écrasement ou repoussement.

10. Cavité cotyloïde artificielle vissable selon une ou plusieurs revendications précédentes comprenant un profil de dent de filetage basculé vers le pôle de la cavité, le flanc côté pôle (**12**) de la dent de filetage (**C**) étant incliné avec un angle (α) par delà le plan radial vers le pôle de cavité.

Fig.1

Fig.2

Fig.3

EP 0 898 470 B1

Fig. 4

Fig. 5